# EUROPEAN PATENT APPLICATION

(11) **EP 1 736 508 A1**
(43) Date of publication of application: **27.12.2006**
(21) Application number: 05013404.8
(22) Date of filing: 22.06.2005
(51) Int. Cl.: C08L 33/02, C08L 101/14, C08L 51/02, A61L 15/60, C08K 3/36, C08K 9/06, C08K 9/04

(54) **Hydrogel-forming polymers with increased permeability and high absorption capacity**

(71) Applicant: BASF Aktiengesellschaft, 67056 Ludwigshafen (DE)
(72) Inventor: Herfert, Norbert, Dr., 63674 Altenstadt (DE); Adey, Vernon L., Hamilton, MS 39746 (US); Carrico, Peter W., West Point, MS 39773 (US); Chiang, William G-J., Yorktown, VA 23692 (US)

(57) **Abstract**

The present invention concerns Hydrogel-forming polymers with increased permeability and high absorption capacity, a process for preparing such Hydrogel-forming polymers and also their use.

## Description

The present invention concerns Hydrugel-forming polymers with increased permeability and high absorption capacity, a process for preparing such Hydrogel-forming polymers and also their use.

Further embodiments of the present invention are disclosed in the claims, the description and the examples. It will be appreciated that the aforementioned features of the present invention and the features of the present invention which will be described hereinbelow can be used not only in the particular combination stated, but also in other combinations without departing from the scope of the present invention.

Hydrogel-forming polymers are swellable and known as superabsorbent polymers (SAPs) or superabsorbents for short, are known from the prior art.

Swellable hydrogel-forming polymers are in particular polymers of (co)polymerized hydrophilic monomers with crosslinking comonomers, graft (co)polymers of one or more hydrophilic monomers on a suitable grafting base, crosslinked cellulose ethers, crosslinked starch ethers, crosslinked carboxymethylcellulose, partially crosslinked polyalkylene oxide or natural products which are capable of swelling in aqueous fluids, such as guar derivatives for example. Such hydrogels are used as products capable of absorbing aqueous solutions to manufacture diapers, tampons, sanitary napkins and other hygiene articles, but also as water-retaining agents in market gardening or to thicken all kinds of wastes, especially medical wastes.

Swellable hydrogel-forming polymers are preferably capable of absorbing at least 10 times their own weight and preferably 20 times their own weight, based on the polymer used, of 0.9% by weight sodium chloride solution. This absorption is preferably achieved even under a pressure of 0.7 psi for example.

Swellable hydrogel-forming polymers are typically surface or gel postcrosslinked to improve their performance characteristics.

This postcrosslinking is known per se to one skilled in the art and preferably takes place in an aqueous gel phase or as surface postcrosslinking of the ground and classified polymeric particles.

Conventional superabsorbents are optimized to absorb urine in hygiene articles, especially baby diapers. Modern baby diapers, but also fem-hygiene articles as well as adult incontinence articles trend to become more thin.

It is therefore extremely advantageous to provide a superabsorbent, which is capable of absorbing large amounts of liquid and shows a high permeability.

The present invention therefore had for its object to develop a specific superabsorbent which is optimally suitable for high absorbancy and increased permeability.

We have found that this object is achieved, surprisingly, by the present invention's addition one or more hydrophobic compounds to the monomer solution or to the polymerized wet gel or to both. It is preferable to add the hydrophobic compounds to the monomer solution before or preferred in the polymerization equipment like a vessel, kneader or polymerization band. Useful hydrophobic compounds are in particular hydrophobicized pyrogenic silicas or hydrophobicized mixtures of pyrogenic silicas and pygrogenic aluminas. Other useful hydrophobic compounds are in particular hydrophobicized precipitated silicas or hydrophobicized mixtures of precipitated silicas and precipitated aluminas. If in the following description pyrogenic silicas or pyrogenic aluminas are mentioned, it is to be understood that also precipitated silicas or precipitated aluminas can be used.

Useful superabsorbent materials in the present invention's process are hydrogels or hydrogel mixtures. The water content of the hydrogels is preferably less than 20% by weight, more preferably less than 10% by weight and most preferably less than 5% by weight. Dried Hydrogel-forming polymer (dried superabsorbent material) is such where the water content is 0%. For example a SAP with 15weight-% water contains 85 weight-percent dry SAP.

The terms "hydrophobic" and "hydrophilic" describe the wetting behavior of a surface with water. The contact angle of a water droplet is smaller than 90° on hydrophobic surfaces, preferred smaller than 80°, more preferred smaller than 70°, specially preferred smaller than 60° and most preferred smaller than 50°. The contact angle is described for example in Colloid Polym. Sci., volume 259 (1981), pages 391 to 394. On rough surfaces the apparent contact angle will be measured. If powders are measured, they shall be smoothed out by a smooth stemp with a pressure of 0.5 bar.

For example, there are free hydroxyl groups on the particle surface of pyrogenic silicas. These hydroxyl groups are capable of forming hydrogen bonds. Pyrogenic silicas are hydrophilic as a result.

Reaction of pyrogenic silicas with trimethylchlorosilane for example can be used to convert the free hydroxyl groups into silyl ether groups. The silyl ether groups are no longer capable of forming hydrogen bonds. The pyrogenic silica has been rendered hydrophobic.

The amounts of hydrophobic particles are advantageously chosen such that not only an increased rate of permeability of the superabsorbents is achieved, but also the process of manufacture of the superabsorbents is has not to be altered substantially.

The amount of hydrophobic particles used is to be individually adjusted with regard of the recipe used in the production.

The present invention provides a process for preparing a Hydroget-forming polymer, , comprising the steps of:
- addition of one or more hydrophobic compounds to the monomer solution or
- addition of one or more hydrophobic compounds to the polymerized wet gel or
- addition of one or more hydrophobic compounds to the monomer solution and to the polymerized wet gel

The hydrophobic compounds are preferably used in the form of particles. The average particle size is typically in the range from 0.001 to 10 µm, preferably in the range from 0.002 to 5 µm, more preferably in the range from 0.005 to 1 µm and most preferably in the range from 0.01 to 0.1 µm. The method of measurement for the particle size distribution is based on the analysis of diffraction spectra according to Fraunhofer. The analyses are standardly carried out using a Mastersizer S, a laser instrument from Malvern. Particular preference is given to polymethylmethacrylats, hydrophobic aluminas, hydrophobic silicas or mixtures thereof. Very particular preference is given to mixtures of hydrophobic silicas with greater than 0% to 20% by weight of hydrophobic alumina and also to hydrophobic silicas. The average primary particle size is preferably in the range from 5 to 50 nm and more preferably in the range from 10 to 20 nm, and the specific surface area is preferably in the range from 10 to 1000 m²/g and more preferably in the range from 80 to 380 m²/g. The amounts used are typically in the range from 0.005% to 20% by weight, preferably in the range from 0.05% to 10% by weight, more preferably in the range from 0.1% to 5% by weight and most preferably less than 1% by weight of hydrophobic, hydrophobicized or hydrophilic compound based on the dry absorbent hydrogel. Preferred hydrophobic compounds are: hydrophobic and/or hydrophobicized clay minerals, hydrophobicized aluminas and/or hydrophobicized silicas, more preferably hydrophobicized aluminas and/or hydrophobicized silicas such as for example Aerosil® R 812, Aerosil® R 974 or Aerosil® R 8200, Sipernat D17® (Degussa Aktiengesellschaft, Germany).

Hydrophobicized aluminas and/or silicas are obtainable for example by reaction of hydrophilic aluminas and/or silicas with hexamethyldisilazane or dimethyldichlorosilane.

The hydrophobic compounds are preferably mixed with the monomer solution before polymerizing to obtain the water-absorbing hydrogel. The hydrophobic compounds can also be mixed with the resulting wet gel. Wet gel means in the context of this invention a hydrogel-forming polymer with an water content of more than 1 part water per part dry polymer, preferable more than 2 parts, more preferable more than 5 parts, most preferable more than 10 parts water per part dry polymer. The wet gel can contain as much water as it can absorb without any pressure. Additional water to the wet gel does not interfere with the possibility to process wet gel with hydrophobic compounds according to this invention. The type of mixing is not subject to any restrictions. It can be done in meat-choppers or other machines know to the person skilled in the art of mixing. Alternatively it is possible to use reaction mixers or mixing and drying ranges, such as for example Lödige® mixers, BEPEX® mixers. NAUTA® mixers, SCHUGGI® mixers, NARA® dryers and PROCESSALL®. Fluidized bed dryers can also be used moreover. The mixing is advantageously carried out using a residence time from 1 to 180 minutes, preferably from 2 to 20 minutes and more preferably from 5 to 20 minutes and a speed from 10 to 1000 rpm, preferably from 50 to 300 rpm and more preferably from 50 to 250 rpm.

The invention further provides a process for aftertreating absorbent hydrogels. The aftertreating can be done before, after or simultaneously to the postcrosslinking step. The aftertreating of absorbent hydrogels comprises the steps of:
- aftertreating with at least one multivalent metal ions, solutions of multivalent metal ions, water-soluble cationic polymers and/or solutions of water-soluble polymers, preferably multivalent metal ions, such as for example Al³⁺, Fe²⁺, Fe³⁺, Ti³⁺, Ti⁴⁺, Co²⁺, Ni²⁺, Cr³⁺, Mn²⁺, Zn²⁺, Mg²⁺, Ca²⁺, Zr³⁺, Zr⁴⁺. more preferably Al³⁺.
- aftertreating with at least one hydrophilic or hydrophobic compound, preferably hydrophobic and/or hydrophobicized clay minerals, hydrophobicized aluminas and/or hydrophobicized silicas, more preferably hydrophobicized aluminas and/or hydrophobicized silicas, such as for example Aerosil® R 812, Aerosil® R 974 or Aerosil® R 8200 (Degussa Aktiengesellschaft, Germany).
- if appropriate aftertreating with at least one anionic, cationic and/or nonionic surfactant, preferably a nonionic surfactant, such as for example sorbitan esters having an HLB value from 2 to 18, particular preference being given to Span® 80 (Uniqema, Netherlands).

The counterions to the multivalent metal ions are not subject to any restriction, but when a solvent is used there is a preference for counterions which ensure adequate solubility, preference being given to sulfate. The metal ions are preferably metered as a solution. Water is a particularly preferred solvent. The concentration of the multivalent metal ion in the aqueous solution is typically from 1% to 20% by weight and preferably from 2% to 10% by weight.

The amount of the multivalent metal ion used is typically in the range from 0.05% to 4% by weight, preferably in the range from 0.1% to 2% by weight and more preferably in the range from 0.2% to 1% by weight based on the absorbing hydrogel.

The surfactants are preferably metered as a solution. Water or a mixture of water and propylene glycol is a particularly preferred solvent. The concentration of the surfactant in the solution is typically in the range from 5% to 70% by weight, preferably in the range from 10% to 50% by weight and more preferably in the range from 20% to 30% by weight.

The amount of surfactant used is typically in the range from 0.01 % to 4% by weight, preferably in the range from 0.05% to 2% by weight and more preferably in the range from 0.1% to 1% by weight based on the absorbing hydrogel.

The order in which the aftertreating agents are metered is not subject to any restriction, but preference is given to the order
- multivalent metal ions, solutions of multivalent metal ions, water-soluble cationic polymers and/or solutions of water-soluble polymers,
- hydrophobic or hydrophilic compounds together or separately in any order,
- surface-active compounds, such as surfactants for example, and also solutions thereof,

preferably only multivalent metal ions and hydrophobic compounds being used for the aftertreatment.

The dissolved aftertreating agents are preferably sprayed onto the dried water-absorbing hydrogel and mixed. The type of mixing is not subject to any restrictions, but preference is given to using reaction mixers or mixing and drying ranges, such as for example Lödige® mixers, BEPEX® mixers, NAUTA® mixers, SCHUGGI® mixers, NARA® dryers and PROCESSALL®. Fluidized bed dryers can also be used moreover. The mixing is advantageously carried out using a residence time from 1 to 180 minutes and preferably from 2 to 15 minutes and a speed from 10 to 1000 rpm, preferably from 50 to 300 rpm and more preferably from 50 to 250 rpm.

The last step may be followed by drying. Drying may take place in the mixer itself, by heating the jacket or introducing a stream of warm air. It is similarly possible to use a downstream dryer, such as for example a tray dryer, a rotary tube oven or a heatable screw. But it is also possible for example to utilize an azeotropic distillation as a drying process.

Preferred drying temperatures in the process of the present invention are in the range from 50 to 250°C, preferably in the range from 50 to 200°C and more preferably in the range from 50 to 150°C. The residence time at this temperature in the reaction mixer or dryer is advantageously below 30 minutes and preferably below 10 minutes.

The drying is preferably carried out at reduced pressure, preferably at less than 500 mbar and more preferably at less than 200 mbar and, if appropriate, supported by a dry gas stream, preferably nitrogen, in an amount from 20 to 1000 l/kgh and preferably from 100 to 250 l/kgh.

Preferably, the absorbent hydrogels are additionally aftertreated with a hydrophilic organic compound in the process of the present invention. Hydrophilic organic compounds improve the fixing of the particulate aftertreating agents on the superabsorbent. Useful hydrophilic organic compounds include for example lower water-soluble polyols having an average molecular weight in the range from 100 to 6000 g/mol, preferably in the range from 200 to 3000 g/mol and more preferably in the range from 250 to 1000 g/mol.

Preferred hydrophilic organic compounds are dendritic polymers, highly branched polymers such as for example polyglycerols, polyethylene glycols, polypropylene glycols, random or block copolymers of ethylene oxide and propylene oxide. Useful compounds for this purpose further include polyethoxylates or polypropoxylates of polyhydroxy compounds, such as glycerol, sorbitol, trimethylolpropane, trimethylolethane, pentaerythritol. Examples thereof are n-tuply ethoxylated trimethylolpropane or glycerol, n being an integer between 1 and 100. Further examples are block copolymers of fully n-tuply ethoxylated and then m-tuply propoxylated trimethylolpropane or glycerol, n being an integer between 1 and 40 and m being an integer between 1 and 40. The order of the blocks can also be the other way around.

The hydrophilic organic compound can be added before, during or after each of the aftertreating steps, preferably before the aftertreatment with the hydrophobic organic compound and more preferably together with the multivalent metal ion.

The hydrophilic organic compound is liquid at 23°C and has a 23°C viscosity of less than 3000 mPas, preferably less than 1500 mPas, more preferably less than 1000 mPas, even more preferably less than 500 mPas and most preferably less than 300 mPas.

The hydrophilic organic compound is used in an amount based on the dried hydrogel that is in the range from 0.01% to 2% by weight, preferably in the range from 0.1% to 1% by weight and more preferably in the range from 0.35% to 0.75% by weight.

The invention further provides crosslinked water-absorbing polymers which, for example, are obtainable by the process of the present invention, especially absorbing hydrogels having an absorbance under load of 0.9psi of at least 10 g/g, preferably of at least 14 g/g, more preferably of at least 16 g/g, even more preferably of at least 18 g/g and particularly preferably of at least 20 g/g, a FS GBP of at least 50 Darcies, preferably of at least 65 Darcies, more preferably of at least 75 Darcies, even more preferably of at least 100 Darcies and particularly preferably of at least 140 Darcies.

The present invention further provides hygiene articles comprising the superabsorbent of the present invention.

The swellable hydrogel-forming polymers which can be used in the process of the present invention are in particular polymers of crosslinked (co)polymerized hydrophilic monomers, polyaspartic acid, graft (co)polymers of one or more hydrophilic monomers on a suitable grafting base, crosslinked cellulose ethers, crosslinked starch ethers or natural products which are swellable in aqueous fluids, such as guar derivatives for example. Preferably, the polymer to be crosslinked is a polymer which comprises structure units which derive from acrylic acid or esters thereof or which were obtained by graft copolymerization of acrylic acid or acrylic esters on a water-soluble polymeric matrix. These hydrogels will be known to one skilled in the art and are described for example in US 4,286,082, DE-C 27 06 135, US 4,340,706, DE-C 37 13 601, DE-C 28 40 010, DE-A 43 44 548, DE-A 40 20 780, DE-A 40 15 085, DE-A 39 17 846, DE-A 38 07 289, DE-A 35 33 337, DE-A 35 03 458, DE-A 42 44 548, DE-A 42 19 607, DE-A 40 21 847, DE-A 38 31 261, DE-A 35 11 086, DE-A 31 18 172, DE-A 30 28 043, DE-A 44 18 881, EP-A 0 801 483. EP-A 455 985, EP-A 467 073, EP-A 312 952, EP-A 205 874, EP-A 499 774, DE-A 26 12 846, DE-A 40 20 780, EP-A 205 674, US 5,145,906, EP-A 0 530 438, EP-A 670 073, US 4,057,521, US 4,062,817, US 4,525,527, US 4,295,987, US 5,011,892, US 4,076.663 or US 4,931,497.

Examples of hydrophilic monomers suitable for preparing these swellable hydrogel-forming polymers are acids which are capable of addition polymerization, such as acrylic acid, methacrylic acid, vinylsulfonic acid, vinylphosphonic acid, maleic acid, maleic anhydride, fumaric acid, itaconic acid, 2-acrylamido-2-methylpropanesulfonic acid, 2-acrytamidv-2-methylpropanephosphonic acid and also their amides, hydroxyalkyl esters and amino- or ammonio-containing esters and amides and also the alkali metal and/or ammonium salts of the acid-functional monomers. It is further possible to use water-soluble N-vinylamides such as N-vinylformamide or else diallyidimethylammonium chloride. Preferred hydrophilic monomers are compounds of the general formula I 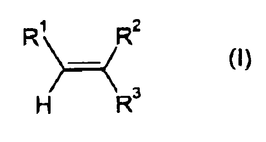 where
- R¹: is hydrogen, methyl, ethyl or carboxyl,
- R²: is -COOR⁴, hydroxysulfonyl or phosphonyl, a phosphonyl group esterified with a C₁-C₄-alkanol, or a group of the formula II

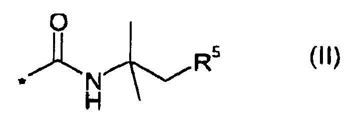

- R³: is hydrogen, methyl or ethyl,
- R⁴: is hydrogen, C₁―C₄-aminoalkyl, C₁―C₄-hydroxyalkyl, alkali metal ion or ammonium ion, and
- R⁵: is a sulfonyl group, a phosphonyl group or a carboxyl group or a respective alkali metal or ammonium salt.

Examples of C₁-C₄-alkanols are methanol, ethanol, n-propanol, isopropanol or n-butanol.

Particularly preferred hydrophilic monomers are acrylic acid and methacrylic acid and also their alkali metal or ammonium salts, for example sodium acrylate, potassium acrylate or ammonium acrylate.

Suitable grafting bases for hydrophilic hydrogels which are obtainable by graft copolymerization of olefinically unsaturated acids or their alkali metal or ammonium salts can be of natural or synthetic origin. Examples are starch, cellulose or cellulose derivatives and also other polysaccharides and oligosaccharides, polyalkylene oxides, especially polyethylene oxides and polypropylene oxides, and also hydrophilic polyesters.

Suitable polyalkylene oxides have for example the formula III 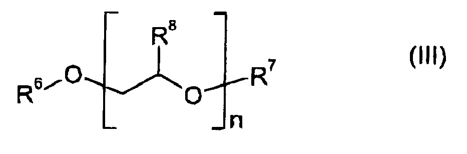 where
- R⁶, R⁷: are independently hydrogen, alkyl, alkenyl or aryl,
- R⁸: is hydrogen or methyl, and
- n: is an integer from 1 to 10 000.
- R⁶ and R⁷: are each preferably hydrogen, C₁-C₄-alkyl, C₂-C₆―alkenyl or phenyl.

Preferred hydrogels are in particular polyacrylates, polymethacrylates and also the US 4,931,497, US 5,011,892 and US 5,041,496 graft polymers.

The swellable hydrogel-forming polymers have preferably been crosslinked, i.e., they comprise compounds having at least two double bonds which have been polymerized into the polymeric network. Suitable crosslinkers are in particular N,N'-methylenebisacrylamide and N,N'-methylenebismethacrylamide, esters of unsaturated mono- or polycarboxylic acids of polyols, such as diacrylate or triacrylate, for example butanediol or ethylene glycol diacrylate or methacrylate and also trimethylolpropane triacrylate and allyl compounds such as allyl (meth)acrylate, triallyl cyanurate, diallyl maleate, polyallyl esters, totraallyloxyethane, triallylamine, tetraallylethylenediamine, allyl esters of phosphoric acid and also vinylphosphonic acid derivatives as described for example in EP-A 343 427. The process of the present invention can further utilize hydrogels which are prepared using polyallyl ethers as a crosslinker and by acidic homopolymerization of acrylic acid. Suitable crosslinkers are pentaerythritol triallyl and tetraallyl ethers, polyethylene glycol diallyl ether, ethylene glycol diallyl ether, glycerol dially ether, glycerol triallyl ether, polyallyl ethers based on sorbitol, and also ethoxylated variants thereof.

The preferred methods of making the base polymer which can be used in the process of the present invention are described in "Modern Superabsorbent Polymer Technology", F.L. Buchholz and A.T. Graham, Wiley-VCH, 1998, pages 77 to 84. Particular preference is given to base polymers which are prepared in a kneader, as described for example in WO 01/38402, or on a belt reactor, as described for example in EP-A 955 086.

The water-absorbing polymer is preferably a polymeric acrylic acid or a polyacrylate. This water-absorbing polymer can be prepared by a process known from the literature. Preference is given to polymers which comprise crosslinking comonomers in amounts from 0.001 to 10 mol% and preferably 0.01 to 1 mol%, but very particular preference is given to polymers which were obtained by free-radical polymerization and where a polyfunctional ethylenically unsaturated free-radical crosslinker was used which additionally bears at least one free hydroxyl group (such as for example pentaerythritol triallyl ether or trimethylolpropane diallyl ether).

The swellable hydrogel-forming polymers are preparable by addition polymerization processes known per se. Preference is given to addition polymerization in aqueous solution conducted as a gel polymerization. It involves for example 15% to 50% by weight aqueous solutions of one or more hydrophilic monomers and if appropriate of a suitable grafting base being addition polymerized in the presence of a free-radical initiator by utilizing the Trommsdorff-Norrish effect (Makromol. Chem. 1, 169 (1947)), preferably without mechanical mixing. The addition polymerization reaction may be carried out in the temperature range between 0 and 150°C and preferably between 10 and 100°C, not only at atmospheric pressure but also at superatmospheric or reduced pressure. As usual, the polymerization can also be carried out in a protective gas atmosphere, preferably under nitrogen. The addition polymerization may be induced using high-energy electromagnetic rays or the customary chemical addition polymerization initiators, for example organic peroxides, such as benzoyl peroxide, tert-butyl hydroperoxide, methyl ethyl ketone peroxide, cumene hydroperoxide, azo compounds such as azodiisobutyronitrile and also inorganic peroxo compounds such as (NH₄)₂S₂O₈ or K₂S₂O₈ or H₂O₂. They may be used if appropriate in combination with reducing agents such as sodium hydrogensulfite and iron(II) sulfate or redox systems, where the reducing component included is an aliphatic or aromatic sulfinic acid, such as benzenesulfinic acid and toluenesuffinic acid or derivatives of these acids, such as Mannich adducts of sulfinic acids, aldehydes and amino compounds, as described in DE-A 13 01 566. The performance characteristics of the polymers can be further improved by postheating the polymer gels in the temperature range from 50 to 130°C and preferably from 70 to 100°C for several hours.

The gels obtained are neutralized for example to 0 to 100 mol% preferably 25 to 80 mol% and more preferably to 50 to 75 mol%, based on monomer used, for which the customary neutralizing agents can be used, preferably alkali metal hydroxides or ammonium hydroxide, alkali metal oxides alkali metal carbonates, ammonium carbonate and alkali metal bicarbonates, but more preferably sodium hydroxide, sodium carbonate and sodium bicarbonate. Neutralization is typically achieved by mixing the neutralizing agent to the monomer solution before polymerization.

Alternatively neutralization is achieved by mixing the neutralizing agent as an aqueous solution or else preferably as a solid into the gel. For this, the gel is mechanically comminuted, for example by means of a meat grinder, and the neutralizing agent is sprayed on, scattered on or poured on and then carefully mixed in. The gel mass obtained can then be repeatedly passed through the meat grinder for homogenization. The neutralized gel mass is then dried with a belt or can dryer until the residual moisture content is preferably below 10% by weight and especially below 5% by weight. The dried hydrogel is subsequently ground and sieved, and the grinding can typically be carried out using roll mills, pin mills or swing mills. The particle size of the sieved hydrogel is preferably in the range from 45 to 1000 µm, more preferably in the range from 45 to 850 µm, even more preferably in the range from 100 to 800 µm and yet more preferably in the range from 100 to 700 µm

The postcrosslinking of hydrogels and superabsorbents is typically carried out by spraying a solution of the surface postcrosslinker onto the dry base polymer powder. After spraying, the polymeric powder is thermally dried, and the crosslinking reaction can take place not only before but also during the drying.

The spraying with a solution of the crosslinker is preferably carried out in reaction mixers or mixing and drying ranges, such as for example Lödige® mixers, BEPEX® mixers, NAUTA® mixers, SCHUGGI® mixers, NARA® dryers and PROCESSALL®. Fluidized bed dryers can be used as well in addition.

Drying may take place in the mixer itself, by heating the jacket or introducing a stream of warm air. It is similarly possible to use a downstream dryer, such as for example a tray dryer, a rotary tube oven or a heatable screw. But it is also possible for example to utilize an azeotropic distillation as a drying process.

Preferred drying temperatures are in the range from 50 to 250°C, preferably in the range from 50 to 200°C and more preferably in the range from 50 to 150°C. The preferred residence time at this temperature in the reaction mixer or dryer is below 30 minutes and more preferably below 10 minutes.

The crosslinker is preferably dissolved in solvents which are not self-reactive, preferably in lower alcohols, such as for example methanol, ethanol, propanediol, ethylene glycol, most preferably in aqueous solutions of such suitable alcohols, in which case the alcohol content of the solution is in the range from 10% to 90% by weight and more preferably in the range from 40% to 60% by weight.

The crosslinker is used in an amount from 0.01% to 1% by weight, based on the polymer used, and the crosslinker solution itself is used in an amount from 1% to 20% by weight and preferably from 5% to 15% by weight, based on the polymer used.

The AUL 0.9psi value [g/g] of the postcrosslinked water-absorbing polymers of the present invention is preferably greater than 10, especially greater than 15, more preferably greater than 20, especially greater than 25 and especially preferably greater than 30.

The present invention's aftertreated hydrogels capable of absorbing aqueous fluids are particularly useful for thickening aqueous solutions and/or suspensions, preferably for thickening aqueous waste solutions and/or waste suspensions, such as for example medical and/or radioactive wastes, most preferably for thickening medical waste solutions and/or waste suspensions.

The present invention's aftertreated hydrogels capable of absorbing aqueous fluids are also useful for absorbing blood and/or body fluids in hygiene articles, such as for example incontinence articles, napkins, tampons, liners. To this end, the present invention's aftertreated hydrogels capable of absorbing aqueous fluids can be processed with fibers, such as cellulose for example, and also fibrous web to form absorbing composites.

An aftertreatment with hydrophobic and hydrophilic materials ensures a uniform distribution of the superabsorbent in the container when treating at least partially liquid wastes, especially at least partially liquid medical wastes, in that at least a portion of the superabsorbent will initially float on the surface of the solution.

The present invention further provides mixtures of products prepared by the present invention's process with biocidal, antimicrobial and/or antibacterial materials and/or perfume or scent materials, stabilizers, dyes, pH indicators and/or other auxiliaries.

To determine the quality of the present invention's aftertreatment, the dried hydrogel is tested by test methods described hereinbelow:

### Methods:

The methods refer if not indicated to the contrary to Edana methods of 2002, Edana is the EUROPEAN DISPOSABLES AND NONWOVENS ASSOCIATION at Avenue Eugène Plasky, 157 - 1030 Brussels - Belgium - www.edana.org
Particle size distribution is measured according to Edana 420.2-02.
Moisture content (dryness) is measured according to Edana 430.2-02.
Free swell capacity is measured according to Edana 440.2-02.
Centrifuge retention capacity (CRC) is measured according to Edana 441.2-02. Absorption under pressure (AUL) is measured according to Edana 442.2-02 and is adopted accordingly for different pressure.

Free-Swell Gel Bed Permeability (FS-GBP): The procedure is disclosed in U.S. Patent 6,387,495 which is incorporated herein by refence in a manner that is consistent herewith. The results are expressed in Darcies.

0.3 psi Gel Bed Permeability (GBP 0.3 psi): The procedure is disclosed in U.S. Patentapplication 10/631916 which is incorporated herein by refence in a manner that is consistent herewith. The results are expressed in Darcies.

### Examples

### Examples 1:

A SAP base polymer was produced by polymerizing a monomer solution containing 26.0 weight-% acrylic acid, 0.50 weight-% based on acrylic acid of pentaerytrittriallyester (P30), the resulting gel was neutralized to a neutralization degree of 72% by using 50 weight-% NaOH.

The polymerization was basically performed as follows:

Transfer 1040 g of glacial acrylic acid into 4L size resin kettle. Add 5.20 g of P-30 crosslinker into acrylic acid solution follow by adding 500 g of DI ice. Add 2430 g of DI water into 4L resin kettle. The acrylic acid mixture solution temperature in resin kettle is about 7~8°C. Start N2 purge at 10 SCFH for 20 minutes. At this point the acrylic acid solution is raise to - 10°C. Add 11.44 g of 1% ascorbic acid and 11.44 g of 1% hydrogen peroxide initiators pair with the syringes. When batch thickening occurs (30 seconds to 10 minutes), remove N2 tube, shut off nitrogen and seal resin kettle with rubber stoppers. Temperature will rise as reaction proceeds. After desired hold time, disassemble equipment and remove gel from resin kettle. Tear and cut gel into smaller pieces. Pass gel pieces through gel chopper one at a time. Add 323 g of 50% NaOH into gel and chop once.

Add no Sipernat D-17 (D-17) in Exp. 1, 1.56 g of D-17 (Exp. 2), 3.12 g of D-17 (Exp. 3), 1.56 g of 22S (Exp.4) or 3.12 g of 22S (Exp,5) into gel and chop once. Disolves 0.40 g of SBS in 200 g water and add into the gel and chop twice. The gel then dried at drum dryer, mill and seive to 850~165 micron. The CRC was measured.

The dryed base polymer was postcrosslinked 4.0weight-% based on SAP of a 3.75 weight% of Denacol solution in 1:1 propyleneglycol:water at 120°C for 1 hour. The CRC, 0.9psi AUL, FSGBP and 0.3 psi GBP were measured.

### Examples 2-5:

Example 1 was repeated, but different Silica were added to the monomer solution:

| | Example 2 | Example 3 | Example 4 | Example 5 |
|---|---|---|---|---|
| Wt-% Sipernat D-17 Silica (hydrophobic silica) each based on acrylic acid | 0,15 | 0,30 | 0,00 | 0,00 |
| Wt-% Sipernat 22-S Silica (hydrophobic silica) each based on acrylic acid | 0,00 | 0,00 | 0,15 | 0,30 |

The results are summarized in the table, which follows:

| base Polymer | Example 1 | Example 2 | Example 3 | Example 4 | Example 5 |
|---|---|---|---|---|---|
| CRC (850-160µ) | 43,6 | 41,4 | 43,3 | 44,9 | 42,5 |

| Postcrosslinked polymer | Example 1 | Example 2 | Example 3 | Example 4 | Example 5 |
|---|---|---|---|---|---|
| CRC (600-300µ) (g/g) | 29,7 | 29,6 | 29,2 | 29,2 | 29,1 |
| 0.9 AUL (g/g) | 21,1 | 20,1 | 20,9 | 20,4 | 19,2 |
| FS GBP (Darcies) | 45,1 | 65,1 | 74,4 | 44,4 | 44,1 |
| 0.3 psi GBP (Darcies) | 1,08 | 1,24 | 1,41 | 0,96 | 0,85 |

### Examples 6 -10:

Examples 1 to 5 were repeated with the addition of 0.14 weight percent based on acrylic acid Kymene 736 to the monomer solution. Kymene 736 is a polyamine-epichlorohydrin.

The results are summarized in the table, which follows:

| | Example 6 | Example 7 | Example 8 | Example 9 | Example 10 |
|---|---|---|---|---|---|
| % D-17 Silica, boAA | 0,00 | 0,15 | 0,30 | 0,00 | 0,00 |
| % 22S Silica, boAA | 0,00 | 0,00 | 0,00 | 0,15 | 0,30 |

| base Polymer | Example 6 | Example 7 | Example 8 | Example 9 | Example 10 |
|---|---|---|---|---|---|
| CRC (850-160µ) | 32,6 | 32,1 | 31,5 | 33,2 | 32,5 |

| Postcrosslinked polymer | Example 6 | Example 7 | Example 8 | Example 9 | Example 10 |
|---|---|---|---|---|---|
| CRC (600-300µ) (g/g) | 29,3 | 28,8 | 28,5 | 28,1 | 28,1 |
| 0.9 AUL (glg) | 20,7 | 20,8 | 20,5 | 18,3 | 18,2 |
| FS GBP (Darcies) | 62 | 75 | 80 | 53 | 49 |

### Example 11 & 12: Base Polymer Synthesis with Pre-Neutralized Polymerization:

The process is a high solid (>50%), low degree of neutralization (<60%) and pre-neutralization process:

Add water to the mix tank. Add glacial acrylic acid into the water. Add the bulk crosslinker into acrylic acid solution and mix thoroughly. Na₂CO₃ (or any other neutralent such as potassium or ammonium carbonates or bicarbonates or combinations of these with hydroxides) slowly added to acrylic acid aqueous solution until completely dissolved, while maintaining the temperature below 50°C. Until you reach the desired degree of neutralization. Maintain an oxygen sparge on the tank during the neutralization process to prevent accidental premature initiation. Heat monomer mixture to desired "Run-to-Belt" temperature. Incorporate 0-50% fines recycle into the monomer stream with a high-shear RM mixer (Philadelphia, Lightnin, or Arde-Barinco for ex.). Hydrophobic Silica (Sipernat D17) should be added separately at this point to the fines recycle stream. It also can be pre-blended with the fines recycle stream depended on equipment set-up. At this point you can add the thermal initiators (V-50 and sodium persulfate)and or ½ of the redox initiator package (FF6 and hydrogen peroxide). Feed the mixture onto suitable polybelt reaction surface while incorporating the balance of the initiator package (FF6 and hydrogen peroxide). Allow monomer mixture to react and polymerize. Allow polymer slab to cool and become conditioned in order to tear into dryable chunks. Pass through a suitable direct or non-direct contact dryer (fluid bed dryer (Carrier, for example), thermal screw dryer (Nara, Bepex, or Holoflite for example) to reduce residual moisture content. Mill (Fitz-mills, cage mills, roll-mills, for example)and sized (Rotex, Minox, Kason for example) to the desired particle size range.

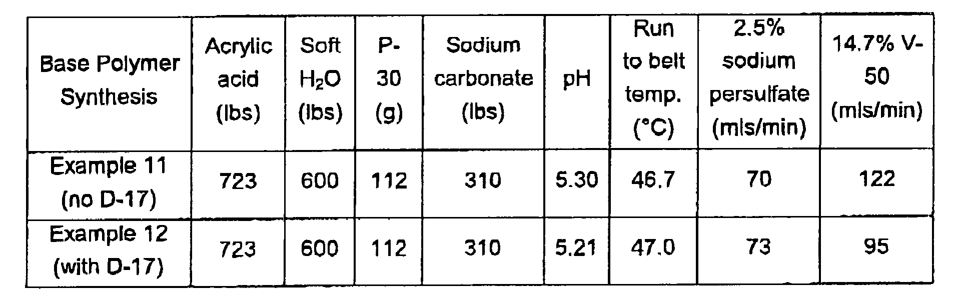

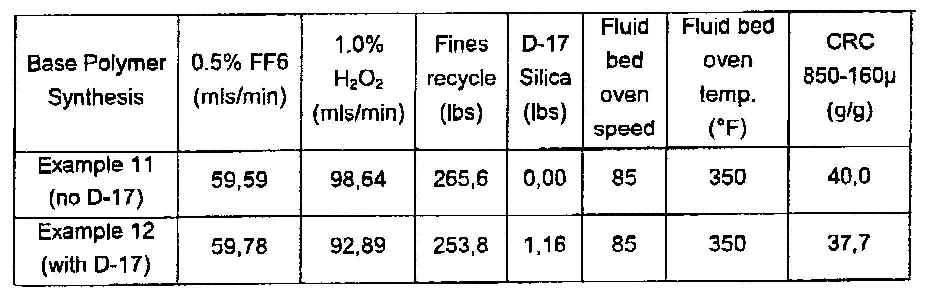

### Procedures of Surface Cross-linking on Base Polymers of examples 11 and 12:

### Examples 13 to 17: Surface Gross-linking on Base Polymer from Example 11 (no D-17 addition):

Surface cross-linking was carried out by Lödige mixer ― 1kg scale. Add 0.15 wt-% of Sipemat 22S to base polymer and mix it in Lödige mixer at 212 rpm for 5 minutes. Surface cross-linking solution:
Solvent solution: Propanediol (PDO):water = 25%:75%
Denacol EX-810: 0.08% - 0.50 wt-% based on polymer
Coating ratio: 4 wt-% based on polymer
Aluminum sulfate: 1 wt-% dry based on polymer or 3.58 wt-% Alum solution.
Curing and drying condition: Dry at lab oven at 135°C for 60 minutes.

| Surface Crosslinked Product from Base Polymer Example 11 | % Denacol | PDO/H₂O | % Al₂(SO₄)₃ | CRC (g/g) | 0.9 psi AUL (g/g) | FS GBP (Darcies) |
|---|---|---|---|---|---|---|
| Example 13 | 0,08 | 25:75 | 1 | 30.2 | 15.5 | 21.2 |
| Example 14 | 0.10 | 25:75 | 1 | 29.4 | 16.1 | 21.8 |
| Example 15 | 0.20 | 25:75 | 1 | 27.7 | 16.1 | 25.5 |
| Example 16 | 0.40 | 25:75 | 1 | 26.2 | 15.2 | 19.3 |
| Example 17 | 0.50 | 25:75 | 1 | 25.8 | 15.4 | 18.3 |

### Examples 18 to 20: Surface Cross-linking on Base Polymer from Example 12 (with D-17 addition):

The procedure is same as Examples 13-17, but Denacol level is from 0.08% ~ 0.12 wt-% based on polymer. The high FS GBP surface cross-linked polymers are yielded in Examples 18-20.

| Surface Crosslinked Product from Base Polymer Example 12 | % Donacol | PDO/H₂O | % Al₂(SO₄)₃ | CRC (g/g) | 0.9 psi AUL (g/g) | FS GBP (Darcies) |
|---|---|---|---|---|---|---|
| Example 18 | 0.08 | 25:75 | 1 | 26.7 | 18.5 | 63 |
| Example 19 | 0.10 | 25:75 | 1 | 26.3 | 17.4 | 73 |
| Example 20 | 0.12 | 25:75 | 1 | 24.8 | 17.3 | 105 |

## Claims

1. A process for preparing a hydrogel-forming polymer, which comprises addition of one or more hydrophobic compounds to the monomer solution or to the polymerized wet gel or to both.

2. A process according to claim 1, wherein the hydrophobic compounds are added to the monomer solution.

3. A process according to claim 1 or 2, wherein the hydrophobic compounds are particles having an average diameter from 0.001 to 10 µm.

4. A process according to any one of claims 1 to 3, wherein the concentration of the hydrophobic compounds is 0.005% to 20% based on the total weight of the dried Hydrogel-forming polymer.

5. A process according to any one of claims 1 to 4. wherein the hydrophobic compounds are hydrophobicized silicas or hydrophobicized mixtures of silicas and aluminas.

6. A process according to any one of claims 1 to 5, wherein the wet gel and/or the hydrogel-forming polymer is additionally aftertreated with a multivalent cation and if appropriate a surfactant.

7. A process according to claim 6, wherein the multivalent cation is an aluminum ion.

8. A process according to claim 6 or 7, wherein the surfactant is a sorbitan ester.

9. A process according to any one of claims 6 to 8, wherein the multivalent cation is metered as an aqueous solution and the surfactant has an HLB value of less than 18.

10. A process according to any one of claims 6 to 9, wherein the aftertreated wet gel and/or the hydrogel-forming polymer is dried.

11. A process according to any one of claims 1 to 10, wherein the hydrogel-forming polymer is dried and postcrosslinked.

12. A hydrogel-forming polymer obtainable a process comprising process steps of any of the claims 1 to 11.

13. The use of the hydrogel-forming polymer according to claim 12 in hygiene articles.

14. Hygiene articles comprising a hydrogel-forming polymer of claim 12.
